# EUROPEAN PATENT SPECIFICATION

(11) **EP 3 703 777 B1**
(45) Date of publication and mention of the grant of the patent: **03.07.2024**
(21) Application number: 18801212.4
(22) Date of filing: 29.10.2018
(51) Int. Cl.: A61M 1/28, A61M 1/16

(54) **DEXTROSE CONCENTRATE FOR THE DIALYSATE AND FOR DISINFECTING**
DEXTROSEKONZENTRAT FÜR DAS DIALYSAT UND ZUM DESINFEKTIEREN
CONCENTRÉ DE DEXTROSE POUR LE DIALYSAT ET POUR DÉSINFECTER

(30) Priority: 30.10.2017 US 201762578841 P
(43) Date of publication of application: 09.09.2020
(73) Proprietor: Baxter International, Inc., Deerfield, IL 60015 (US); Baxter Healthcare SA, 8152 Glattpark (Opfikon) (CH)
(72) Inventor: MATHIOT, Sarah Lynn, Lake Zurich Illinois 60047 (US); WELLINGS, Anders J., Belleair Beach Florida 33786 (US); COOK, Jeffrey, Lake Barrington Illinois 60010 (US); SZPARA, Edward, Saint Charles Illinois 60175 (US); WIESLANDER, Anders, 227 38 Lund (SE); STRAKA, Paul, Park Ridge Illinois 60068 (US)
(74) Representative: Potter Clarkson
(86) International application number: PCT/US2018/057977
(87) International publication number: WO 2019/089447

(56) References cited:
- EP-B1- 2 714 123
- WO-A1-2012/163737
- DE-A1-102009 038 213

## Description

### BACKGROUND

The present disclosure relates generally to medical fluid devices. More specifically, the present disclosure relates to medical fluid devices that mix fluid online for treatment or that receive fluid mixed online for treatment. The invention relates specifically to renal failure therapy systems.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is life saving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion.

Hemofiltration ("HF") is an alternative renal replacement therapy that relies on a convective transport of toxins from the patient's blood. HF is accomplished by adding substitution or replacement fluid to the extracorporeal circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, providing convective clearance.

Most HD, HF, and HDF treatments occur in centers. A trend towards home hemodialysis ("HHD") exists today in part because HHD can be performed daily, offering therapeutic benefits over in-center hemodialysis treatments, which occur typically bi- or triweekly. Studies have shown that more frequent treatments remove more toxins and waste products and render less interdialytic fluid overload than a patient receiving less frequent but perhaps longer treatments. A patient receiving more frequent treatments does not experience as much of a down cycle (swings in fluids and toxins) as does an in-center patient, who has built-up two or three day's worth of toxins prior to a treatment. In certain areas, the closest dialysis center can be many miles from the patient's home, causing door-to-door treatment time to consume a large portion of the day. Treatments in centers close to the patient's home may also consume a large portion of the patient's day. HHD can take place overnight or during the day while the patient relaxes, works or is otherwise productive.

Another type of kidney failure therapy is peritoneal dialysis ("PD"), which infuses a dialysis solution, also called dialysis fluid, into a patient's peritoneal cavity via a catheter. The dialysis fluid is in contact with the peritoneal membrane in the patient's peritoneal cavity. Waste, toxins and excess water pass from the patient's bloodstream, through the capillaries in the peritoneal membrane, and into the dialysis fluid due to diffusion and osmosis, i.e., an osmotic gradient occurs across the membrane. An osmotic agent in the PD dialysis fluid provides the osmotic gradient. Used or spent dialysis fluid is drained from the patient, removing waste, toxins and excess water from the patient. This cycle is repeated, e.g., multiple times.

There are various types of peritoneal dialysis therapies, including continuous ambulatory peritoneal dialysis ("CAPD"), automated peritoneal dialysis ("APD"), tidal flow dialysis and continuous flow peritoneal dialysis ("CFPD"). CAPD is a manual dialysis treatment. Here, the patient manually connects an implanted catheter to a drain to allow used or spent dialysis fluid to drain from the peritoneal cavity. The patient then switches fluid communication so that the patient catheter communicates with a bag of fresh dialysis fluid to infuse the fresh dialysis fluid through the catheter and into the patient. The patient disconnects the catheter from the fresh dialysis fluid bag and allows the dialysis fluid to dwell within the peritoneal cavity, wherein the transfer of waste, toxins and excess water takes place. After a dwell period, the patient repeats the manual dialysis procedure, for example, four times per day. Manual peritoneal dialysis requires a significant amount of time and effort from the patient, leaving ample room for improvement.

Automated peritoneal dialysis ("APD") is similar to CAPD in that the dialysis treatment includes drain, fill and dwell cycles. APD machines, however, perform the cycles automatically, typically while the patient sleeps. APD machines free patients from having to manually perform the treatment cycles and from having to transport supplies during the day. APD machines connect fluidly to an implanted catheter, to a source or bag of fresh dialysis fluid and to a fluid drain. APD machines pump fresh dialysis fluid from a dialysis fluid source, through the catheter and into the patient's peritoneal cavity. APD machines also allow for the dialysis fluid to dwell within the cavity and for the transfer of waste, toxins and excess water to take place. The source may include multiple liters of dialysis fluid including several solution bags.

APD machines pump used or spent dialysate from the peritoneal cavity, though the catheter, and to the drain. As with the manual process, several drain, fill and dwell cycles occur during dialysis. A "last fill" may occur at the end of the APD treatment. The last fill fluid may remain in the peritoneal cavity of the patient until the start of the next treatment, or may be manually emptied at some point during the day.

In any of the above modalities using an automated machine, the automated machine operates typically with a disposable set, which is discarded after a single use. Depending upon the complexity of the disposable set, the cost of daily the disposable may become significant. Also, daily disposables require space for storage, which can become a nuisance for home owners and businesses. Moreover, daily disposable replacement requires daily setup time and effort by the patient or caregiver at home or at a clinic.

For each of the above reasons, extended use disposables for dialysis treatments are needed.

WO 2012/163737 discloses a blood treatment apparatus that is adapted to preserve a blood treatment unit between blood treatment sessions. The blood treatment apparatus is configured to (i) perform a blood treatment session, (ii) fill the blood treatment unit with a preservation fluid comprising at least one treatment fluid concentrate of a type that is used to prepare the treatment fluid, (iii) maintain the preservation fluid in the blood treatment unit until a next blood treatment session is prepared, (iv) dispatch the preservation fluid from the blood treatment unit in preparation for a next blood treatment session, and (v) perform a next blood treatment session.

### SUMMARY

A first aspect of the present invention provides a renal failure therapy system according to claim 1. A second aspect of the present invention provides a renal failure therapy system according to claim 14.

The examples described herein disclose automated systems and methods applicable, for example, to fluid delivery for: peritoneal dialysis ("PD"), plasmapherisis, hemodialysis ("HD"), hemofiltration ("HF") hemodiafiltration ("HDF"), continuous renal replacement therapy ("CRRT"), apheresis, autotransfusion, hemofiltration for sepsis, and extracorporeal membrane oxygenation ("ECMO") treatments. The systems and methods described herein are applicable to any medical fluid delivery system in which the treatment fluid may be made online or at the point of use, e.g., just before and/or during treatment. These modalities may be referred to collectively or generally individually herein as medical fluid delivery system(s).

Moreover, each of the systems and methods described herein may be used with clinical or home-based treatments. For example, the present systems and methods may be employed in in-center PD, HD, HF or HDF machines, which run throughout the day. Alternatively, the present systems and methods may be used with home PD, HD, HF or HDF machines, which are operated generally at the patient's convenience.

In one embodiment, a peritoneal dialysis system is provided having point of use dialysis fluid production in combination with an extended use disposable. The system includes a cycler and a water purifier. The cycler includes a control unit having at least one processor and at least one memory. The cycler may further include a wired or wireless transceiver for sending information to and receiving information from the water purifier. The water purifier may also include a control unit having at least one processor and at least one memory and a wired or wireless transceiver for sending information to and receiving information from the control unit of the cycler.

The cycler includes equipment programmed via its control unit to prepare fresh dialysis solution at the point of use, pump the freshly prepared dialysis fluid to a patient, allow the dialysis fluid to dwell within the patient, then pump used dialysis fluid to a drain. The above cycles are then repeated over the course of treatment. The cycler in one embodiment includes a heater under control of the control unit for heating the dialysis fluid as it is being mixed, so that dialysis fluid delivered to the patient is at least approximately at body temperature, e.g., about 37°C. The heater may for example be located at the top of a housing of the cycler, e.g., beneath a heating lid.

The system may run a PD therapy that after the multiple fill, dwell and drain cycles described, provides a "last bag" fill of dialysis fluid as a last patient fill before the patient disconnects from the PD cycler. The last fill remains with the patient until the patient performs a manual drain or reconnects to the cycler for a new treatment. The last fill dialysis fluid is formulated differently than the online dialysis fluid made from the concentrate and used for the other fills and for the disinfection of the disposable set. It is accordingly contemplated in one embodiment to provide a new last fill dialysis fluid bag for each treatment needing same or to provide a last bag with enough last bag formulated dialysis fluid to last for multiple, e.g., all, extended use treatments using the same disposable set.

The cycler (and the water purifier in one embodiment) operates with a disposable set. The disposable set in one embodiment includes a disposable cassette, which may include a planar rigid plastic piece covered on one or both sides by a flexible membrane, forming fluid pumping and valving chambers. In one example, fluid pump chambers may operate with pneumatic pump chambers of the cycler, while fluid valve chambers operate with pneumatic valve chambers of the cycler. In other examples, the pump and valve actuation may be electromechanical, e.g., peristaltic for pump actuation and solenoid pinch clamp for valve actuation.

The disposable set may include (i) a patient line that extends from the cassette to a patient line connector, (ii) a drain line that extends from the cassette to a drain line connector (which may in turn connect removeably to the water purifier), (iii) a heater/mixing line that extends from the cassette to a heater/mixing bag of the present disclosure, (iv) an upstream water line segment that extends from the water purifier to a water inlet of a water accumulator and a downstream water line segment that extends from a water outlet of the water accumulator to the cassette, (v) a last bag or sample line that extends from the cassette to a premixed last fill bag of dialysis fluid or to a sample bag or other sample collecting container, (vi) a first, e.g., dextrose, concentrate line extending from the cassette to a first, e.g., dextrose, concentrate container, and optionally (vii) a second, e.g., buffer, concentrate line that extends from the cassette to a second, e.g., buffer, concentrate container.

In the system and method of the present disclosure, the control unit is further programmed to pump one of the concentrates used to make peritoneal dialysis fluid into the disposable set to disinfect the set between uses. The dextrose concentrate and the buffer concentrate may both be acidic and have average pH values lower than 7.0. Dextrose concentrate is more acidic than buffer concentrate. Dextrose is used accordingly for reuse in one preferred embodiment. It is believed that the disinfecting power of the concentrates, and in particular dextrose concentrate, is due to one or both of the low pH of the concentrate and/or the lower water activity of the concentrate. For example 50% or 70% dextrose concentrate solution may yield a water activity that helps to prevent the spread of bacteria and other organisms (yeast, mold, etc.). (See, e.g., United States Pharmacopeial Convention ("USP") <1112> Application of Water Activity/General Information). That in combination with the acidic properties of the concentrates may result in their disinfecting ability. It is possible, however, that low pH alone produces the disinfecting properties of the concentrates.

It is contemplated to use the dextrose concentrate in at least two ways. The first way is to use the dextrose concentrate prior to treatment to disinfect the disposable set. In one embodiment, after connection of the disposable set to the concentrate bags and the water purifier, the system flushes the disposable set with dextrose concentrate before the start of treatment. If any touch contamination has occurred during connection of the disposable set to the solution/concentrate bags and/or water device, flushing the set with the dextrose concentrate increases the likelihood that the bacteria and other organisms will be killed by the dextrose concentrate, thereby potentially reducing the chance of peritonitis.

The second use of dextrose is performed after treatment. After completion of treatment and patient disconnection from the disposable set, the system fills the set (does not have to be a complete or total fill) with dextrose concentrate solution to disinfect the set for the next treatment. The dextrose concentrate remains in the disposable set until the next treatment, allowing plenty of time for disinfection. Before the next treatment, the dextrose concentrate solution is pumped out of the disposable set. The emptied disposable set is then disinfected for reuse. The system then uses a same supply of the dextrose concentrate to create dialysis fluid for the next treatment.

In an embodiment, the dextrose concentrate container is sized for example to hold three treatment volumes and two disinfection volumes worth of dextrose, such that the disposable set may be used for three treatments with two disinfections in between. Disposable cost, space consumed, and setup time and effort here are reduced by two-thirds. The dextrose concentration container may be on the order of four to six liters. The buffer container may be smaller because extra buffer is not needed for disinfection and may be on the order of three liters. Both concentrate containers are replaced at the same time in one method, e.g., after three treatments.

The disposable set in an embodiment includes a disposable cassette and multiple tubes extending from the disposable cassette. During disinfection, most of the tubes remain connected to whatever they are connected to during treatment. For example, the concentrate tubes remain connected to their respective concentrate containers, the water line remains connected to the water purifier, and the drain line remains connected to a drain connector of the water purifier. In an alternative embodiment, the water line and drain line may be removed from the water purifier and connected together.

The patient line however is disconnected from the patient after treatment and needs to be handled properly during disinfection. In one embodiment, the patient line is connected to a spare port on the disposable cassette of the disposable set. In another embodiment, the patient line is connected to a port provided by the drain line, e.g., via a Y-connector provided in the drain line. In a further embodiment, the patient line is capped via a disinfectant cap. The cap may be provided with a hydrophobic vent so that dextrose may be pumped readily through the entire patient line, pushing air out of the hydrophobic vent.

The disposable cassette is left connected to the peritoneal dialysis cycler during disinfection in one embodiment, so that the cycler may pump dextrose concentrate wherever it needs to reach. In an embodiment, the control unit of the cycler is programmed to cause the disposable cassette to pump dextrose throughout the disposable cassette, to fill the patient line with dextrose, to wet all interior surfaces of the heater/mixing bag, and to fill at least part of the drain line, which may thereafter be clamped closed in the water purifier. The heater/mixing bag may be evacuated after treatment to compress the bag, reducing the volume of dextrose needed (e.g., to a liter or less) to contact all interior surfaces of the bag.

The disposable set may include a water accumulator to hold water made suitable for dialysis. It is believed that the water accumulator does not need to be disinfected between treatments. The buffer concentrate container line can be filled with buffer concentrate line and likewise does not need to be filled with dextrose.

During the disinfection phase, it is contemplated to have the cycler agitate the dextrose and perhaps heat the dextrose to aid its disinfection effect. It is contemplated to use a dextrose concentrate that is fifty percent dextrose by volume. Upon starting the next treatment, it is contemplated to have the cycler rinse all dextrose to drain using water made suitable for dialysis.

The user interface of the cycler in one embodiment provides a timer that shows the user how long the disinfection fluid or dextrose has resided within the disposable set. The system also includes a conductivity sensor that provides feedback to the control unit of the cycler, confirming that a concentrate such as dextrose has actually been distributed to the disposable set. The conductivity sensor may be located along the drain line.

The renal failure therapy system of the first aspect includes: a dialysis fluid pumping unit including a dialysis fluid pump; a disposable set operable with the dialysis fluid pumping unit such that the dialysis fluid pump can pump dialysis fluid from the disposable set; a concentrate in fluid communication with the disposable set, wherein the concentrate is used to prepare the dialysis fluid; and a control unit operating the dialysis fluid pump, the control unit configured to (i) cause a portion of the concentrate to fill at least a portion of the disposable set between treatments, the concentrate operating as a disinfectant allowing the same disposable set to be used for multiple treatments with the dialysis pumping unit and (ii) determine whether the disposable set may be used again via the disinfecting concentrate in (i).

In a first embodiment which may be combined with the first aspect, the control unit is configured to cause the dialysis fluid pump to pump the portion of the concentrate to fill the at least the portion of the disposable set between treatments.

In a second embodiment which may be combined with the first aspect or the first embodiment, the renal failure therapy system is a peritoneal dialysis system and the dialysis pumping unit is a peritoneal dialysis cycler.

In a third embodiment which may be combined with the first aspect or any one of the first and second embodiments, the at least a portion of the disposable set holding the concentrate between treatments incudes a pumping cassette operable with the dialysis fluid pump and at least one line in fluid communication with the pumping cassette.

In a fourth embodiment which may be combined with the first aspect or any one of the first to third embodiments, the concentrate includes dextrose.

In a fifth embodiment which may be combined with the first aspect or any one of the first to fourth embodiments, the concentrate is acidic.

In a sixth embodiment which may be combined with the first aspect or any one of the first to fifth embodiments, the renal failure therapy system includes a supply of water made suitable for treatment, and wherein the control unit is configured to mix the concentrate with the water made suitable for treatment to form the dialysis fluid.

In a seventh embodiment which may be combined with the sixth embodiment, the disposable set incudes a container for accumulating water made suitable for treatment, and wherein the accumulating container does not receive the concentrate between treatments.

In an eighth embodiment which may be combined with the sixth or seventh embodiment, the concentrate is a first concentrate and which includes a second concentrate, and wherein the control unit is configured to mix the first and second concentrates with the water made suitable for treatment to form the dialysis fluid.

In a ninth embodiment which may be combined with the eighth embodiment, the second concentrate is used to disinfect a second concentrate line of the disposable set between treatments.

In a tenth embodiment which may be combined with the first aspect or any one of the first to ninth embodiments, the concentrate is provided in a first container in an amount such that after use of the concentrate to prepare dialysis fluid for treatment, enough concentrate remains to fill the at least the portion of the disposable set for disinfection, and wherein a second container of concentrate is used for a subsequent treatment.

In an eleventh embodiment which may be combined with the first aspect or any one of the first to tenth embodiments, the concentrate is provided in a container in an amount such that after use of the concentrate to prepare dialysis fluid for treatment, enough concentrate remains to fill the at least the portion of the disposable set for disinfection and to prepare dialysis fluid for a subsequent treatment.

In a twelfth embodiment which may be combined with the first aspect or any one of the first to eleventh embodiments, the control unit is configured to cause the dialysis fluid pump to remove the concentrate from the at least the portion of the disposable set prior to preparing dialysis fluid for a subsequent treatment.

In a thirteenth embodiment which may be combined with the first aspect or any one of the first to twelfth embodiments, the removed concentrate is replaced with water made suitable for treatment.

In a fourteenth embodiment which may be combined with the first aspect or any one of the first to thirteenth embodiments, the disposable set includes a heater/mixing bag, and wherein the control unit is programmed to cause the heater/mixing bag to collapse prior to introducing the concentrate into the heater/mixing bag for disinfection.

In a fifteenth embodiment which may be combined with the third embodiment, the disposable set includes a patient line, and wherein (i) the disposable cassette is configured to connect to a distal end of the patient line between treatments or (ii) a cap is provided to cap the distal end of the patient line between treatments.

In a sixteenth embodiment which may be combined with the first aspect or any one of the first to fifteenth embodiments, the renal failure therapy system incudes a last bag of dialysis fluid formulated differently than dialysis fluid made from the concentrate used for disinfection, and wherein the last bag is provided with enough last bag dialysis fluid for a single treatment or the last bag is provided with enough last bag dialysis fluid for multiple treatments using the same disposable set.

The renal failure therapy system of the second aspect includes: a dialysis fluid pumping unit including a dialysis fluid pump; a disposable set operable with the dialysis fluid pumping unit such that the dialysis fluid pump can pump dialysis fluid from the disposable set; a concentrate in fluid communication with the disposable set, wherein the concentrate is used to prepare the dialysis fluid; and a control unit operating the dialysis fluid pump, the control unit configured to (i) cause a portion of the concentrate to fill at least a portion of the disposable set prior to treatment, the concentrate operating as a disinfectant to disinfect the at least portion of the disposable set prior to treatment and (ii) determine whether the disposable set may be used again via the disinfecting concentrate in (i).

Also described herein but not claimed is a renal failure therapy method that includes: mixing water made suitable for dialysis with at least one concentrate to form a dialysis fluid; moving the dialysis fluid through a disposable set to perform a dialysis treatment creating used dialysis fluid; removing the used dialysis fluid through the disposable set; and disinfecting at least a portion of the disposable set using a concentrate of the at least one concentrate.

In one method, the moving and removing occur multiple times before the disinfecting.

Another method includes moving a last bag fill of dialysis fluid through the disposable set to perform a dialysis treatment between the removing and the disinfecting.

Another method includes removing a prior delivered concentrate for disinfecting the at least a portion of the disposable set before mixing water made suitable for dialysis with the at least one concentrate.

In light of the present disclosure and the above aspects, it is therefore an advantage of the present disclosure to provide an improved medical fluid system and method.

It is another advantage of the present disclosure to provide a medical fluid system and method that creates medical fluid at the point of use and reuses a disposable component to reduce cost.

It is a further advantage of the present disclosure to provide a medical fluid system and method that creates medical fluid at the point of use and reuses a disposable component to reduce storage space.

It is still another advantage of the present disclosure to provide a medical fluid system and method that creates medical fluid at the point of use and reuses a disposable component to save setup time and effort.

It is still a further advantage of the present disclosure to provide a medical fluid system and method that creates medical fluid at the point of use and reuses a disposable component while maintaining an acceptable level of microbiological hygiene by providing adequate disinfection between treatments.

The advantages discussed herein may be found in one, or some, and perhaps not all of the embodiments disclosed herein. Additional features and advantages are described herein, and will be apparent from, the following Detailed Description and the figures.

### BRIEF DESCRIPTION OF THE FIGURES

Fig. 1 is a front elevation view of one example system and method employing extended use point of care dialysis fluid generation of the present disclosure.
Fig. 2 is a top plan view of one embodiment of a disposable set operable with the system of Fig. 1.
Fig. 3 is a schematic flow diagram for using the extended use of the present disclosure to disinfect a disposable set prior to treatment.
Fig. 4 is a schematic flow diagram for using the extended use of the present disclosure to disinfect a disposable set between treatments.

### DETAILED DESCRIPTION

### System Overview

The examples described herein are applicable to any medical fluid therapy system that delivers a medical fluid that may be mixed at the point of use, prior to and/or during treatment, such as dialysis fluid, substitution fluid, or an intravenous drug. The examples are particularly well suited for kidney failure therapies, such as all forms of peritoneal dialysis ("PD"), hemodialysis ("HD"), hemofiltration ("HF"), hemodiafiltration ("HDF") and continuous renal replacement therapies ("CRRT"), referred to herein collectively or generally individually as renal failure therapy. Moreover, the systems and methods described herein may be used in clinical or home settings. For example, the systems and associated methods may be employed in an in-center PD or HD machine, which runs virtually continuously throughout the day. Alternatively, the systems and methods may be used in a home PD or HD machine, which can for example be run at night while the patient is sleeping. The systems and methods discussed herein are also applicable to medical delivery or intravenous drug applications. The following examples will be described in the setting of a peritoneal dialysis system having extended use point of care dialysis fluid production but may instead be used to make extended use point of care treatment fluid for any of the above modalities.

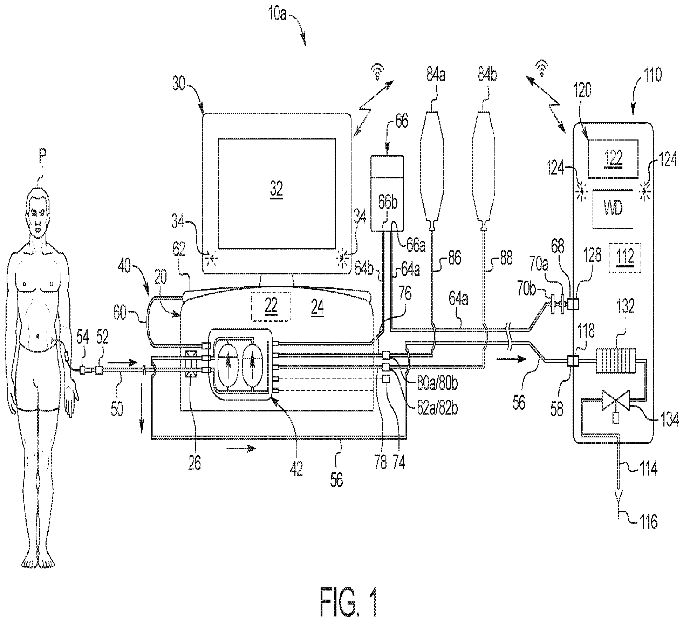

Referring now to the drawings and in particular to Fig. 1, one embodiment of a peritoneal dialysis system having extended use point of care dialysis fluid production of the present disclosure is illustrated by system 10. System 10 includes a cycler 20 and a water purifier 110. Suitable cyclers for cycler 20 include, e.g., the Amia^{®} or HomeChoice^{®} cycler marketed by Baxter International Inc., with the understanding that those cyclers are provided with updated programming to perform and use the point of use dialysis fluid produced according to system 10. To this end, cycler 20 includes a control unit 22 having at least one processor and at least one memory. Control unit 22 further incudes a wired or wireless transceiver for sending information to and receiving information from a water purifier 110. Water purifier 110 also includes a control unit 112 having at least one processor and at least one memory. Control unit 112 further includes a wired or wireless transceiver for sending information to and receiving information from control unit 22 of cycler 20. Wired communication may be via Ethernet connection, for example. Wireless communication may be performed via any of Bluetooth^{™}, WiFi^{™}, Zigbee^{®}, Z-Wave^{®}, wireless Universal Serial Bus ("USB"), or infrared protocols, or via any other suitable wireless communication technology.

Cycler 20 includes a housing 24, which holds equipment programmed via control unit 22 to prepare fresh dialysis solution at the point of use, pump the freshly prepared dialysis fluid to patient P, allow the dialysis fluid to dwell within patient P, then pump used dialysis fluid to a drain. In the illustrated embodiment, water purifier includes a drain line 114 leading to a drain 116, which can be a house drain or a drain container. The equipment programmed via control unit 22 to prepare fresh dialysis solution at the point of use in an embodiment includes equipment for a pneumatic pumping system, including but not limited to (i) one or more positive pressure reservoir, (ii) one or more negative pressure reservoir, (iii) a compressor and a vacuum pump each under control of control unit 22, or a single pump creating both positive and negative pressure under control of control unit 22, to provide positive and negative pressure to be stored at the one or more positive and negative pressure reservoirs, (iv) plural pneumatic valve chambers for delivering positive and negative pressure to plural fluid valve chambers, (v) plural pneumatic pump chambers for delivering positive and negative pressure to plural fluid pump chambers, (vi) plural electrically actuated on/off pneumatic solenoid valves under control of control unit 22 located between the plural pneumatic valve chambers and the positive and negative pressure reservoirs, (vii) plural electrically actuated variable orifice pneumatic valves under control of control unit 22 located between the plural pneumatic pump chambers and the positive and negative pressure reservoirs, (viii) a heater under control of control unit 22 for heating the dialysis fluid as it is being mixed, and (ix) an occluder 26 under control of control unit 22 for closing the patient and drain lines in alarm and other situations.

In one embodiment, the plural pneumatic valve chambers and the plural pneumatic pump chambers are located on a front face or surface of housing 24 of cycler 20. The heater is located inside housing 24 and in an embodiment includes heating coils that contact a heating pan, which is located at the top of housing 24, beneath a heating lid (not seen in Fig. 1).

Cycler 20 in the illustrated embodiment includes a user interface 30. Control unit 22 in an embodiment includes a video controller, which may have its own processing and memory for interacting with primary control processing and memory of control unit 22. User interface 30 includes a video monitor 32, which may operate with a touch screen overlay placed onto video monitor 32 for inputting commands via user interface 30 into control unit 22. User interface 30 may also include one or more electromechanical input device, such as a membrane switch or other button. Control unit 22 may further include an audio controller for playing sound files, such as voice activation commands, at one or more speaker 34.

Water purifier 110 in the illustrated embodiment also includes a user interface 120. Control unit 112 of water purifier 110 in an embodiment includes a video controller, which may have its own processing and memory for interacting with primary control processing and memory of control unit 112. User interface 120 includes a video monitor 122, which may likewise operate with a touch screen overlay placed onto video monitor 122 for inputting commands into control unit 112. User interface 120 may also include one or more electromechanical input device, such as a membrane switch or other button. Control unit 112 may further include an audio controller for playing sound files, such as alarm or alert sounds, at one or more speaker 124 of water purifier 110.

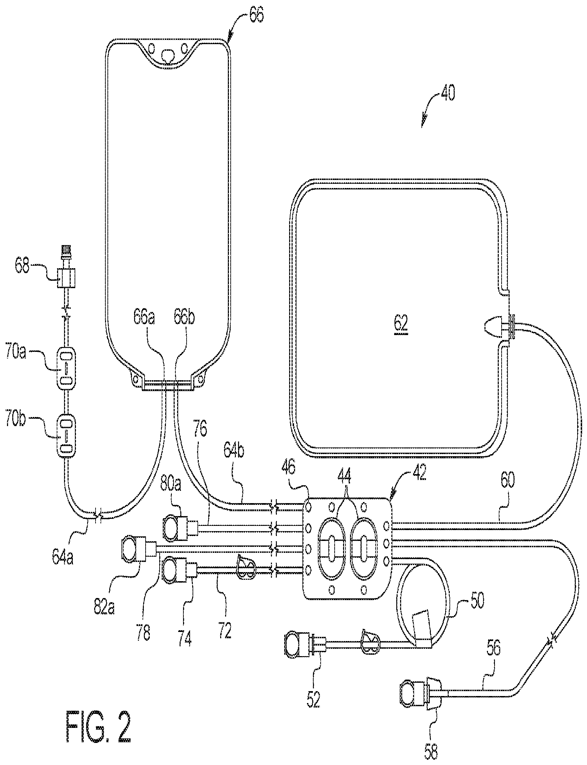

Referring additionally to Fig. 2, one embodiment of disposable set 40 is illustrated. Disposable set 40 is also illustrated in Fig. 1, mated to cycler 20 to move fluid within the disposable set 40, e.g., to mix dialysis fluid as discussed herein. Disposable set 40 in the illustrated embodiment includes a disposable cassette 42, which may include a planar rigid plastic piece covered on one or both sides by a flexible membrane. The membrane pressed against housing 24 of cycler 20 forms a pumping and valving membrane. Fig. 2 illustrates that disposable cassette 42 includes fluid pump chambers 44 that operate with the pneumatic pump chambers located at housing 24 of cycler 20 and fluid valve chambers 46 that operate with the pneumatic valve chambers located at housing 24 of cycler 20.

Figs. 1 and 2 illustrate that disposable set 40 includes a patient line 50 that extends from a patient line port of cassette 42 and terminates at a patient line connector 52. Fig. 1 illustrates that patient line connector 52 connects to a patient transfer set 54, which in turn connects to an indwelling catheter located in the peritoneal cavity of patient P. Disposable set 40 includes a drain line 56 that extends from a drain line port of cassette 42 and terminates at a drain line connector 58. Fig. 1 illustrates that drain line connector 58 connects removeably to a drain connector 118 of water purifier 110. Water purifier 110 includes its own drain line 114 that runs from drain line connector 58 past a conductivity sensor 132 ahead of a solenoid drain valve 134, which is under the control of control unit 112 of the water purifier. In the extended use sequences discussed below, conductivity sensor 132 is used to sense that concentrate has indeed been introduced into disposable set 40 (concentrate has a different conductivity than water). If control unit 112 receives a reading from conductivity sensor 132 indicating that disinfecting concentrate is present, control unit 112 sends an, e.g., wireless, signal to control unit 22 of cycler 20 indicating same. Control unit 22 may then cause user interface 30 to display a timer on video monitor 32 of cycler 20 that shows how long the disinfecting fluid has been disinfecting the disposable set. If no such signal is received at control unit 22 after a period of time in which control unit 22 expects such a signal, control unit 22 may provide an audio, visual or audiovisual alarm at user interface 30 indicating that an issue with disinfection needs to be addressed.

Figs. 1 and 2 further illustrate that disposable set 40 includes a heater/mixing line 60 that extends from a heater/mixing line port of cassette 42 and terminates at a heater/mixing bag 62. Disposable set 40 includes an upstream water line segment 64a that extends to a water inlet 66a of water accumulator 66. A downstream water line segment 64b extends from a water outlet 66b of water accumulator 66 to cassette 42. In the illustrated embodiment, upstream water line segment 64a begins at a water line connector 68 and is located upstream from water accumulator 66. Fig. 1 illustrates that water line connector 68 is removeably connected to a water outlet connector 128 of water purifier 110.

Water purifier 110 outputs water and possibly water suitable for peritoneal dialysis ("WFPD"). To ensure WFPD, however, a sterile sterilizing grade filter 70a is placed upstream from a downstream sterile sterilizing grade filter 70b, respectively. Filters 70a and 70b may be placed in water line segment 64a upstream of water accumulator 66. Sterile sterilizing grade filters 70a and 70b may be pass-through filters that do not have a reject line. Pore sizes for filters 70a and 70b may, for example, be less than a micron. Suitable sterile sterilizing grade filters 70a and 70b may be provided by the assignee of the present disclosure. In an embodiment, only one of upstream or downstream sterilizing filter 70a and 70b is needed to produce WFPD, nevertheless, two sterile sterilizing grade filters 70a and 70b are provided in the illustrated embodiment for redundancy in case one fails.

Fig. 2 further illustrates that a last bag or sample line 72 may be provided that extends from a last bag or sample port of cassette 42. Last bag or sample line 72 terminates at a connector 74, which may be connected to a mating connector of a premixed last fill bag of dialysis fluid or to a sample bag or other sample collecting container. Last bag or sample line 72 and connector 74 may be used alternatively for a third type of concentrate if desired.

Last bag or sample line 72 and connector 74 may also be used after treatment for the extended use disinfection discussed herein, where patient P disconnects patient connector 52 of patient line 50 from transfer set 54 and then reconnects patient connector 52 to connector 74 of line 72, forming a loop between patient line 50 and cassette 42 for a disinfecting concentrate to circulate between treatments to provide disinfection. In an alternative embodiment, drain line 56 is provided with a Y-connector or T-connector having a free port that is normally capped (not illustrated). Here, when patient P disconnects patient connector 52 of patient line 50 from transfer set 54 after treatment, patient P removes the cap from the free port of the Y-connector or T-connector and connects patient connector 52 to the free port. A loop is thereby formed between patient line 50, drain line 56 and cassette 42 for a disinfecting concentrate to circulate between treatments to provide disinfection. In a further alternative, when patient P disconnects patient connector 52 of patient line 50 from transfer set 54 after treatment, patient P applies, e.g., threads, a cap (not illustrated) onto patient connector 52. The cap may be provided with a disinfectant that spreads over patient connector 52 when applied. The cap may also be provided with a hydrophobic vent that vents air out of the patient line 50, allowing an easier flow of concentrate into the patient line for disinfection.

Figs. 1 and 2 illustrate that disposable set 40 includes a first, e.g., dextrose, concentrate line 76 extending from a first concentrate port of cassette 42 and terminates at a first, e.g., dextrose, cassette concentrate connector 80a. A second, e.g., buffer, concentrate line 78 extends from a second concentrate port of cassette 42 and terminates at a second, e.g., buffer, cassette concentrate connector 82a.

Fig. 1 illustrates that a first concentrate container 84a holds a first, e.g., dextrose, concentrate, which is pumped from container 84a through a container line 86 to a first container concentrate connector 80b, which mates with first cassette concentrate connector 80a. A second concentrate container 84b holds a second, e.g., buffer, concentrate, which is pumped from container 84b through a container line 88 to a second container concentrate connector 82b, which mates with second cassette concentrate connector 82a.

As discussed herein, dextrose concentrate is used in one embodiment to disinfect disposable set 40 between treatments. Assuming first concentrate container 84a and container line 86 to hold dextrose and second concentrate container 84b and container line 88 to hold buffer concentrate, it should be appreciated that because buffer concentrate is high in sodium chloride concentration and has high osmolality in low water activity, the buffer concentrate also displays an ability to disinfect, so that dextrose does not need to be introduced into buffer line 88 or buffer container 84b for disinfection. Similarly, it is believed that water accumulator 66 and upstream water line segment 64a do not need to receive disinfecting fluid between treatments. Downstream water line segment 64b may or may not receive disinfecting fluid between treatments. While first and second concentrate containers 84a and 84b are sized in one embodiment to hold enough concentrate for the entire duration of a single disposable set 40 (e.g., 4 to 6 liters of dextrose for three treatments plus two disinfection sessions and 3 liters of buffer for three treatments), it is also possible to replace the concentrate containers after each treatment and associated disinfection.

When a new disposable set 40 is removed from its sterile packaging and used for the first time, patient P (or caregiver) in one procedure loads cassette 42 into cycler 20 and in a random or designated order (i) places heater/mixing bag 62 onto cycler 20, (ii) connects upstream water line segment 64a to water outlet connector 128 of water purifier 110, (iii) connects drain line 56 to drain connector 118 of water purifier 110, (iv) connects first cassette concentrate connector 80a to first container concentrate connector 80b, and (v) connects second cassette concentrate connector 82a to second container concentrate connector 82b. At this point, patient connector 52 is still capped. Once fresh dialysis fluid is prepared and verified, patient line 50 is primed with fresh dialysis fluid, after which patient P may connect patient line connector 52 to transfer set 54 for treatment. Each of the above steps may be illustrated graphically at video monitor 32 and/or be provided via voice guidance from speakers 34.

It is contemplated for the extended use sequences described herein to evacuate heater/mixing bag 62 prior to filling it with disinfecting fluid, e.g., dextrose. To do so, control unit 22 of cycler 20 in an embodiment causes a vacuum to be pulled on the fluid pumping chambers of disposable cassette 42 with all cassette fluid valves closed except the cassette fluid valve to heater/mixing bag 62, causing air to move from the heater/mixing bag 62 to the fluid pump chambers. Next, control unit 22 of cycler 20 causes the cassette fluid valve to heater/mixing bag 62 to close and the cassette fluid valve to drain line 56 to open, and apply a positive pressure to the fluid pump chambers to push air from the chambers to drain. The above cycle is repeated until the heater/mixing bag 62 is fully evacuated, pulling its sheets together. In this manner, the amount of disinfecting fluid needed to wet all internal surfaces of the heater/mixing bag 62 is minimized.

For disposable set 40, the rigid portion of cassette 42 may be made for example of a thermal olefin polymer of amorphous structure ("TOPAS") cyclic olefin copolymer ("coc"). The flexible membranes of cassette 42 may be made for example of a copolyletser ether ("PCCE") and may be of one or more layer. Any of the tubing or lines may be made for example of polyvinyl chloride ("PVC"). Any of the connectors may be made for example of acrylonitrile-butadienestyrene ("ABS", e.g., for the connectors of heater/mixing bag 62, for concentrate connectors 80a, 80b, 82a, 82b and the connectors for any other line of disposable cassette 42, including Y-connectors, T-connectors and caps for any of the lines), acrylic (e.g., for drain line connector 58) or PVC (e.g., for water line connector water line connector 68). Any of the bags or containers, such as heater/mixing bag or container 62 and concentrate bags or containers 84a and 84b may be made of PVC. The materials for any of the above components may be changed over time.

### Concentrate Disinfection

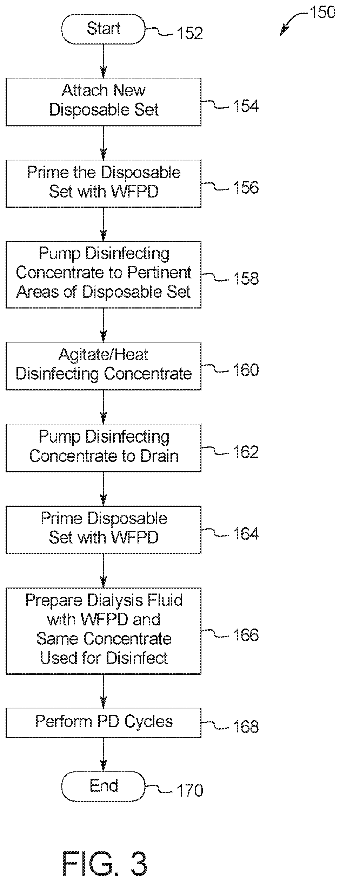

Referring now to Fig. 3, method 150 illustrates one procedure for using concentrate such as dextrose, which is otherwise used to prepare dialysis fluid, to disinfect a disposable item for a dialysis treatment. At oval 152, method 150 begins. At block 154, user interface 30 of cycler 20 prompts patient P to install a new disposable set 40 and walks patient P though setup.

At block 156, control unit 22 causes cycler 20 to prime disposable set 40 with WFPD. WFPD is in one method pumped to cassette 42, patient line 50, heater/mixing line 60 and bag 62, and drain line 56, sending air down drain lines 56 and 114 to drain 116. The concentrate lines leading to cassette 42 are primed using their respective concentrates in one method, but could be primed alternatively with WFPD.

At block 158, control unit 22 causes cycler 20 to pump a disinfecting concentrate, such as dextrose, to pertinent areas of disposable set 40. In one embodiment, the pertinent areas of disposable set 40 include all fluid pathways, pump chambers and valve chambers of cassette 42, patient line 50, heater/mixing line 60, heater/mixing bag 62, at least a portion of drain line 56, and perhaps downstream water line segment 64b. Alternatively, the disinfecting concentrate is pumped to contact cassette 42 and at least a portion of drain line 56, and not to heater/mixing line 60, heater/mixing 62 and patient line 50. One primary goal of the present disclosure is to kill any bacteria or other organisms that may have entered set 40 when the concentrates are connected, which are then flushed to cassette 42 when the concentrate lines are primed using their respective concentrates.

If dextrose is supplied via concentrate container 84a and concentrate line 86, concentrate line 88 will then be disinfected via concentrate, e.g., buffer, via concentrate container 84b. In one method, cassette 42 is wetted first with dextrose concentrate via line 86 for disinfection. The remaining lines may be wetted in any desired order, e.g., (i) buffer concentrate line, (ii) heater/mixing line/bag, (ii) patient line, and (iii) drain line. As discussed above, heater/mixing bag 62 may be collapsed under negative pressure from cycler 20 prior to receiving disinfecting concentrate so as to limit the amount of disinfecting concentrate needed to properly wet the inner surfaces of the heater/mixing bag.

At block 160, control unit 22 causes cycler 20 to perform an optional disinfecting concentrate heating and/or agitation sequence. For example, cycler 20 may cause its fluid heater to heat the disinfecting concentrate within heater/mixing bag 62 and then circulate the heated disinfecting fluid to different desired areas of disposable set 40. Cycler 20 may cause the disinfecting concentrate to reverse directions one or more times to aid the disinfecting concentrate in contacting all needed interior surfaces of disposable set 40.

At block 162, control unit 22 causes cycler 20 to pump the disinfecting concentrate down drain lines 56 and 114 to drain 116. At the end of this sequence, disposable set 40 may be at least substantially dry and disinfected for an upcoming treatment.

At block 164, control unit 22 causes cycler 20 to again prime disposable set 40 with WFPD. WFPD is pumped to cassette 42, patient line 50, heater/mixing line 60 and bag 62, and drain line 56, sending air down drain lines 56 and 114 to drain 116 in one method, which helps to also flush any remnants of the disinfecting fluid also to drain. Priming may be performed alternatively after dialysis fluid made from WFPD is prepared using the dialysis fluid.

At block 166, control unit 22 causes cycler 20 to mix the same disinfecting concentrate, e.g., dextrose and an additional concentrate, e.g., buffer, with WFPD from water accumulator 66 to prepare dialysis fluid for treatment. In an embodiment, conductivity sensor 132 in drain line 56 is used to provide feedback to control unit 22 indicating that the dialysis fluid has been mixed properly for treatment.

At block 168, control unit 22 causes cycler 20 to perform plural drain, fill and dwell cycles (assuming patient P is full initially with the previous day's last fill, otherwise cycle order is fill, dwell and drain). Each of the cycles removes additional patient fluid known as ultrafiltration, which is sent to drain. It is contemplated that heater/mixing bag 62 can hold multiple fill cycles' worth of dialysis fluid, however, cycler 20 may have to mix additional dialysis fluid during treatment. In one method, at the end of the last drain using dialysis fluid made online from the same concentrate used to disinfect disposable set 40 at the beginning of treatment, a last fill of a dialysis solution is provided to patient P. The last fill dialysis fluid is formulated to remain within patient P over a prolonged period of time, e.g., until a day exchange or until the next full treatment.

At block 170, method 150 ends.

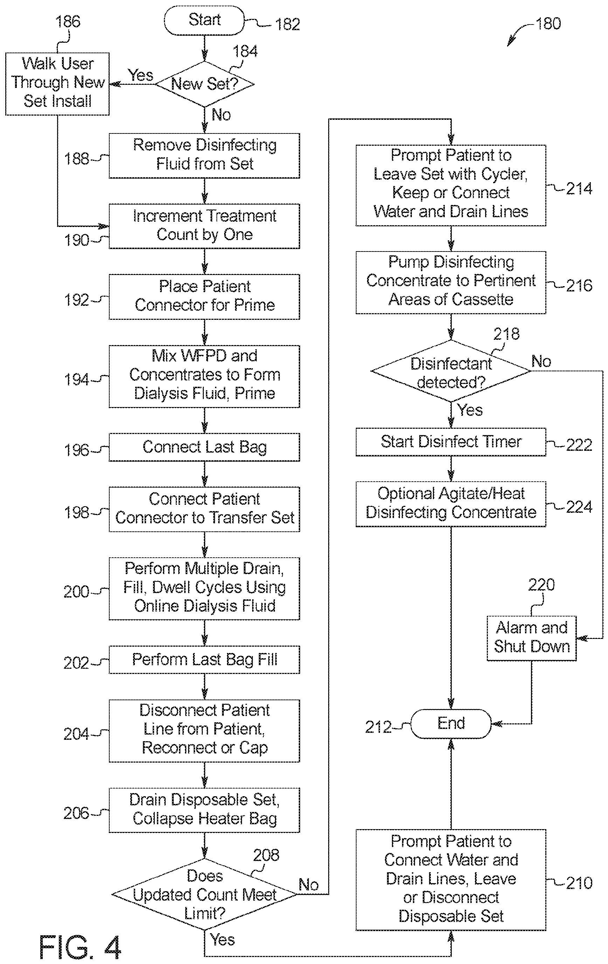

Referring now to Fig. 4, method 180 illustrates one procedure for using concentrate such as dextrose, which is otherwise used to prepare dialysis fluid, to disinfect a disposable item between treatments, so that the disposable item may be used for multiple treatments. At oval 182, method 180 begins. At diamond 184, control unit determines whether a new disposable set 40 needs to be installed. It is contemplated that using the disinfecting concentrate between treatments allows the disposable set to be reused over multiple treatments but not indefinitely. Accordingly, a number of treatments that may be performed using a same disposable set 40 is to be determined either theoretically or empirically. For example, the same disposable set 40 may be used for three to five treatments with a concentrate disinfection between each of the treatments. Control unit 22 counts how many treatments have been performed using the same disposable set 40. At block 184, when the counted number reaches the limit, control unit 22 causes user interface 30 of cycler 20 to request that a new disposable set 40 be installed for the next treatment.

As shown below at diamond 208, user interface 30 may audibly, visually, or audiovisually tell the patient or caregiver to remove the existing set 40 at the end of treatment when the number of treatments limit has been reached and not perform a disinfecting concentrate. Alternatively, if the number of treatments limit has been reached, the used-up disposable set may remain connected to cycler 20 without receiving disinfecting concentrate until the next treatment, where, user interface 30 audibly, visually, or audiovisually tells the patient or caregiver to remove the existing set 40 and install a new disposable set 40. In either case, at block 186, when the number of treatments limit has been reached, user interface 30 in the present treatment prompts the patient or caregiver to install a new disposable set 40 and audibly visually or audiovisually walks the user through the setup steps in one method.

At diamond 184, when the number of treatments for the current disposable set is instead less than the preset limit stored in control unit 22, meaning that at least one additional treatment using the current disposable set may be performed, control unit 22 at block 188 causes cycler 20 to operate disposable cassette 42 prior to treatment to pump the disinfecting concentrate currently residing in disposable set 40 to drain 116 via drain lines 56 and 114. In the one method, control unit 22 of cycler 20 sends a signal, e.g., wireless, to control unit 112 of water purifier 110 telling the water unit to open solenoid drain valve 134, allowing fluid to flow to drain 116 via water purifier drain line 114. Cycler 20 pumps the currently residing disinfecting concentrate, e.g., dextrose, from heater/mixing bag 62, heater/mixing line 60, patient line 50, and dextrose concentrate line 86, through disposable cassette 42, down drain lines 56 and 114 to drain 116. Cycler 20 may also pump existing buffer concentrate from buffer line 86, through disposable cassette 42, down drain lines 56 and 114 to drain 116. At the end of block 188, disposable set 40 should be at least substantially free of the disinfecting concentrate dwelling within in disposable set 40 prior to treatment.

The above paragraph assumes that drain line 56 of disposable set 40 remains connected to water purifier 110 between treatments for disinfection. Alternatively, where drain line 56 is connected instead to upstream water line segment 64a between treatments for disinfection (creating a loop), control unit 22 of cycler at block 188 instead instructs patient P to disconnect drain line 56 from upstream water line segment 64a and to connect both the drain line and the upstream water line segment 64a to water purifier 110. Control unit 22 of cycler 20 may then send the signal to control unit 112 of water purifier 110 telling the water unit to open solenoid drain valve 134, allowing fluid to flow to drain 116 via water purifier drain line 114. Draining of the disinfecting concentrates then proceeds as described above.

At block 190, once the draining of disinfection fluid from disposable set 40 occurs, control unit 22 of cycler 20 increments a number of treatments counter by one. If a new disposable set 40 has instead been installed at cycler 20, control unit will still increment the number of treatments by one because system 10 in an embodiment is operable to fill disposable set 40 with disinfecting concentrate even if treatment is aborted. Once disposable set 40 is removed from its sterile packaging, it is prone to bacteria or other organisms even if treatment is aborted.

At block 192, with a new or disinfected disposable set 40 installed at cycler 20 (e.g., cassette 42 loaded into cycler 20, heater/mixing bag 62 placed onto the heater of cycler 20, upstream water line segment 64a connected to water outlet connector 128 of water purifier 110, drain line 56 connected to drain connector 118 of water purifier 110, first cassette concentrate connector 80a connected to first container concentrate connector 80b, and second cassette concentrate connector 82a connected to second container concentrate connector 82b). Patient P maneuvers patient connector 52 into a position for priming, e.g., against a patient line prime clip/sensor provided by cycler 20. If disposable set 40 is new, patient P simply places the patient connector 52 into a priming clip/sensor on cycler 20 that holds connector 52 at a desired height for priming. If disposable set 40 is reused and patient connector 52 is connected to either cassette 42 or drain line 56, patient P removes the patient connector from the cassette or drain line, places the patient connector 52 into the priming clip/sensor on cycler 20, and caps the exposed port at the cassette or drain line. If disposable set 40 is reused and patient connector 52 is instead capped, patient P may leave the cap in place if vented or remove the cap if not vented and place the patient connector 52 into the priming clip/sensor on cycler 20.

At block 194, control unit 22 causes cycler 20 to mix WFPD with dextrose and buffer concentrates and to pump the mixture back and forth between cassette 42 and heater/mixing bag 62 in a mixing sequence, while heating the mixture at heater/mixing bag 62. When a homogeneous and desired mixture is achieved and verified, e.g., via feedback from conductivity sensor 132 to control unit 22 via water purifier control unit 112, control unit 22 causes cycler to prime any remaining areas of disposable set 40, e.g., patient line 50, which has been positioned for priming, pushing air to heater/mixing bag 62 and/or to drain 116. As discussed above, priming may be performed alternatively using WFPD only, after which dialysis fluid using WFPD is prepared.

At block 196, if a last bag of dialysis solution is to be used, control unit 22 causes user interface 30 to prompt patient P or a caregiver to connect the last bag of dialysis fluid to last bag or sample line 72 of cassette 42. The last bag of dialysis fluid is different physiologically from the dialysis fluid made online in one method, so a new last bag may be used for each new treatment when the last bag is prescribed for the patient. Control unit 22 of cycler 20 opens the valve to last bag or sample line 72 of cassette 42 and pulls last bag solution into line 72 to prime the line, pushing air to heater/mixing bag 62 and/or drain. In an alternative embodiment, the last bag may be sized to hold enough last bag dialysis fluid for multiple, e.g., each, of the extended use treatments, so that patient P switches disposable set 40 and the last bag together at the end of the multiple extended use treatments. In such a case, block 196 is not needed.

At block 198, control unit 22 causes user interface 30 to prompt patient P to connect patient connector 52 and primed patient line 50 to patient transfer set 54 for treatment.

At block 200, control unit 22 causes cycler 20 to perform plural drain, fill and dwell cycles (assuming patient P is full initially with the previous day's last fill, otherwise cycle order is fill, dwell and drain). Each of the cycles removes additional patient fluid known as ultrafiltration, which is sent to drain. It is contemplated that heater/mixing bag 62 can hold multiple fill cycles' worth of dialysis fluid, however, cycler 20 may have to mix additional dialysis fluid during treatment.

At block 202, if a last bag has been designated for patient P's treatment, control unit 22 causes cycler 20 to deliver a last bag fill to the patient. The last bag volume remains with patient P even after disconnection from disposable set 40 until manually drained on the next treatment.

At block 204, control unit 22 causes user interface 30 to prompt patient P to disconnect patient connector 52 from transfer set 54 and to (i) reconnect patient connector 52 to sample line 72 of cassette 42, (ii) reconnect patient connector 52 to drain line 56 or (iii) place a disinfectant cap on patient connector 52.

At block 206, with solenoid drain valve 134 of water purifier 110 open, control unit 22 causes cycler 20 to pump any dialysis fluid, concentrate, and/or water remaining in disposable set 40, through drain lines 56 and 114 to drain 116. Dialysis fluid from heater/mixing bag 62, heater/mixing line 60, patient line 50 and cassette 42 is pumped to drain 116. The draining of heater/mixing bag 62 in one method also collapses the heater/mixing bag under negative pressure from cycler 20. Doing so prior to receiving disinfecting concentrate limits the amount of disinfecting concentrate needed to properly wet the inner surfaces of the heater/mixing bag, e.g., to half the total volume of the bag. Any WFPD remaining in water accumulator 66 and associated lines 64a and 64b is also pumped to drain 116. Buffer in concentrate line 88 may also be removed to drain 116. Alternatively, WFPD remaining in water accumulator 66 and associated lines 64a and 64b and/or buffer in concentrate line 88 may be maintained within disposable set.

In one preferred approach, if it is determined at diamond 208 that the updated number of treatments has reached the limit of treatments, then heater/mixing bag 62 and line 60, concentrate bags 84a and 84b and associated lines, the last fill bag and line 72, and water accumulator 66 and associated lines are drained to empty. If it is determined at diamond 208 that the updated number of treatments has not reached the limit of treatments, and disposable set 40 is to be disinfected, then only heater/mixing bag 62 and line 60 water accumulator 66 and associated lines are drained in one method. It should be noted that methods 170 and 180 are not limited to the steps being performed in the order illustrated and described in Figs. 3 and 4. For example, the determination at diamond 208 may be made well in advance, e.g., at diamond 184.

At diamond 208 and as discussed above, if the updated number of treatments has reached the limit of treatments, control unit 22 at block 210 may cause user interface 30 to prompt patient P to either (i) disconnect upstream water line segment 64a and drain line 56 from water purifier 110, reconnect those lines together at connectors 68 and 58, and remove disposable set 40 from cycler or (ii) disconnect upstream water line segment 64a and drain line 56 from water purifier 110, reconnect those lines together at connectors 68 and 58, but leave disposable set 40 connected to cycler.

At oval 212, method 180 ends.

At diamond 208, if the updated number of treatments has not reached the limit of treatments, control unit 22 at block 214 may cause user interface 30 to prompt patient P to either (i) leave disposable set 40 connected to cycler with water line segment 64a and drain line 56 connected to water purifier 110 or (ii) disconnect upstream water line segment 64a and drain line 56 from water purifier 110, reconnect those lines together at connectors 68 and 58, and leave disposable set 40 connected to cycler.

At block 216, control unit 22 causes cycler 20 to pump a disinfecting concentrate, e.g., dextrose to disposable set 40, including cassette 42, heater/mixing line 60 and bag 62, patient line 50, and drain line 56. With heater/mixing bag 62 collapsed, it is not required to pump the entire full bag volume's worth of disinfectant to contact all inner surfaces of the bag. For example, perhaps only one-half of mixing bag 62's full volume of disinfecting fluid would need to be introduced to contact all inner surfaces of the mixing bag. Cassette 42, heater/mixing line 60 and bag 62, patient line 50, and at least a portion of drain line 56 are in one alternative completely filled with the disinfecting concentrate to perform the disinfection.

At diamond 218 with solenoid drain valve 134 still open, control unit 22 looks for a signal from control unit 112 of water purifier 110 indicating that conductivity sensor 132 ahead of a solenoid drain valve 134 sees the disinfecting concentrate, e.g., dextrose. If the disinfectant detected signal is not received in an expected amount of time, control unit 22 causes user interface 30 at block 220 to provide an audio, visual or audiovisual alarm indicating an issue with disinfection. Method 180 then ends at oval 212.

If the disinfectant detected signal is received within the expected amount of time, control unit 22 at block 222 sends a signal, e.g., wireless, to control unit 112 of water purifier 110 to close solenoid drain valve 134 and begins running a disinfection timer, which may be displayed on user interface 30. At block 224, control unit 22 causes cycler 20 to perform an optional disinfecting concentrate heating and/or agitation sequence. For example, cycler 20 may cause its fluid heater to heat the disinfecting concentrate within heater/mixing bag 62 and then circulate the heated disinfecting fluid to different desired areas of disposable set 40. Cycler 20 alternatively or additionally may cause the disinfecting concentrate to reverse directions one or more times to aid the disinfecting concentrate in contacting all needed interior surfaces of disposable set 40. Method 180 then ends at oval 212.

### Disinfection Data

Testing has been performed to determine the growth rate of bacteria, yeast, and mold spores in pertinent concentrate solutions over prolonged time periods and at room temperature. In the tables below, a log difference of > 1 log indicates growth of the test organisms, a log difference of <1 and > -1 indicates no growth or death of the test organisms, while a log difference of < -1 indicates death of the test organisms. For the buffer concentrate, which has a slight acidic pH of approximately 6.3, most test organisms exhibited no growth or death, with two organisms exhibiting death. For the dextrose concentrate, which has a more acidic pH of 3.5, two test organisms exhibited no growth or death, with all other test organisms dying. As discussed above, the water activity of the concentrates may also play a role in their disinfecting (or organism growth stunting) properties.

| | Time (hrs) | **AB ATCC 16404** | **BD ATCC 19146** | **CA ATCC 10231** | **EC ATCC 25922** | **PA ATCC 27853** | **BC ATCC25416** | **EF ATCC 19433** | **KP ATCC 13883** | **RP ATCC 700590** | **SA ATCC 6538** | **SE ATCC 12228** |
|---|---|---|---|---|---|---|---|---|---|---|---|---|
| N=3 avg | **0** | 3.72 | 3.34 | 3.20 | 4.43 | 3.69 | 2.80 | 3.36 | 2.67 | 3.58 | 3.52 | 2.04 |
| | **24** | 3.63 | 3.34 | 2.41 | 4.15 | 3.48 | 1.83 | 3.20 | 2.28 | 1.36 | 3.41 | 1.52 |
| | **48** | 3.28 | 3.34 | 2.63 | 3.89 | 3.28 | 1.26 | 3.11 | 2.53 | 1.36 | 3.00 | 1.36 |
| | **72** | 3.59 | 3.28 | 2.61 | 3.79 | 2.97 | -0.78 | 2.93 | 2.41 | 0.40 | 2.58 | 1.17 |
| Log change (T=0 to T=72hr) | | -0.13 | -0.06 | -0.59 | -0.64 | -0.72 | -3.58 | -0.43 | -0.26 | -3.18 | -0.94 | -0.87 |

20X Buffer at Room Temperature (20X is one part buffer to 19 parts water)

| | Time (hrs) | **AB ATCC 16404** | **BD ATCC 19146** | **CA ATCC 10231** | **EC ATCC 25922** | **PA ATCC 27853** | **BC ATCC25416** | **EF ATCC 19433** | **KP ATCC 13883** | **RP ATCC 700590** | **SA ATCC 6538** | **SE ATCC 12228** |
|---|---|---|---|---|---|---|---|---|---|---|---|---|
| N=3 avg | **0** | 3.76 | 3.28 | 3.20 | 2.36 | 2.90 | 1.94 | 3.38 | 2.84 | 3.52 | 2.59 | 1.46 |
| | **24** | 3.66 | 0 | 2.99 | 0 | 0 | 1.26 | 0.70 | -0.70 | -1.00 | 0 | 0.76 |
| | **48** | 3.26 | 0 | 2.98 | 0 | 0 | 0 | 0 | -1.00 | -0.70 | 0 | -0.70 |
| | **72** | 3.60 | 0 | 2.83 | 0 | 0 | 0 | 0 | -1.00 | 0 | 0 | 0 |
| Log change (T=0 to T=72hr) | | -0.16 | -3.28 | -0.37 | -2.36 | -2.90 | -1.94 | -3.38 | -3.42 | -3.52 | -2.59 | -1.46 |

50% By Volume Dextrose at Room Temperature

| **Abbreviation** | **Organism Name** | **ATCC** # | **Organism Type** |
|---|---|---|---|
| AB | *Aspergillus brasiliensis* | ATCC 16404 | Mold Spore |
| BC | *Burkholderia cepacia* | ATCC 25416 | Gram Negative Rod |
| BD | *Brevundimons diminuta* | ATCC 19146 | Gram Negative Rod |
| CA | *Candida albicans* | ATCC 10231 | Yeast |
| EC | *Escherichia coli* | ATCC 25922 | Gram Negative Rod |
| EF | *Enterococcus faecalis* | ATCC 19433 | Gram Positive Cocci |
| KP | *Klebisella pneumoniae* | ATCC 13883 | Gram Negative Rod |
| PA | *Pseudomonas aeruginosa* | ATCC 27853 | Gram Negative Rod |
| RP | *Ralstonia pickettii* | ATCC 700590 | Gram Negative Rod |
| SA | *Staphylococcus aureus* | ATCC 6538 | Gram Positive Cocci |
| SE | *Staphylococcus epidermidis* | ATCC 12228 | Gram Positive Cocci |

Bacteria, Yeast, and Mold Spore Abbreviations

It should be understood that various changes and modifications to the presently preferred embodiments described herein will be apparent to those skilled in the art.

## Claims

1. A renal failure therapy system (10) comprising:
a dialysis fluid pumping unit (20) including a dialysis fluid pump;
a disposable set (40) operable with the dialysis fluid pumping unit such that the dialysis fluid pump can pump dialysis fluid from the disposable set;
a concentrate in fluid communication with the disposable set, wherein the concentrate is used to prepare the dialysis fluid;
a conductivity sensor (132) configured to sense when the concentrate has been introduced to the disposable set; and
a control unit (22) operating the dialysis fluid pump,
**characterized in that** the control unit is configured to:
(i) cause a portion of the concentrate to fill at least a portion of the disposable set between treatments, the concentrate operating as a disinfectant allowing the same disposable set to be used for multiple treatments with the dialysis fluid pumping unit,
(ii) use an output of the conductivity sensor to determine whether the concentrate filled the at least a portion of the disposable set,
(iii) when the concentrate did not fill the at least a portion of the disposable set, cause an alarm to be provided that is indicative of an issue with the concentrate, and
(iv) determine whether the disposable set may be used again via the disinfecting concentrate in (i).

2. The renal failure therapy system of Claim 1, wherein the control unit (22) is configured to cause the dialysis fluid pump to pump the portion of the concentrate to fill the at least the portion of the disposable set (40) between treatments.

3. The renal failure therapy system of Claim 1 or 2, wherein the renal failure therapy system is a peritoneal dialysis system and the dialysis fluid pumping unit is a peritoneal dialysis cycler.

4. The renal failure therapy system of any one of Claims 1 to 3, wherein the at least a portion of the disposable set (40) holding the concentrate between treatments includes a pumping cassette (42) operable with the dialysis fluid pump and at least one line (50, 60, 72) in fluid communication with the pumping cassette, wherein the pumping cassette includes a patient line (50) and wherein (i) the pumping cassette is configured to connect to a distal end of the patient line between treatments or (ii) a cap is provided to cap the distal end of the patient line (50) between treatments.

5. The renal failure therapy system of any one of Claims 1 to 4, wherein the concentrate includes dextrose.

6. The renal failure therapy system of any one of Claims 1 to 5, which includes a supply of water made suitable for treatment, and wherein the control unit (22) is configured to mix the concentrate with the water made suitable for treatment to form the dialysis fluid.

7. The renal failure therapy system of Claim 6, wherein the disposable set (40) includes a container (66) for accumulating water made suitable for treatment, and wherein the accumulating container does not receive the concentrate between treatments.

8. The renal failure therapy system of Claim 6 or 7, wherein the concentrate is a first concentrate and which includes a second concentrate, and wherein the control unit (22) is configured to mix the first and second concentrates with the water made suitable for treatment to form the dialysis fluid.

9. The renal failure therapy system of Claim 8, wherein the second concentrate is used to disinfect a second concentrate line (78) of the disposable set (40) between treatments.

10. The renal failure therapy system of any one of Claims 1 to 9, wherein the concentrate is provided in a first container (84a) in an amount such that after use of the concentrate to prepare dialysis fluid for treatment, enough concentrate remains to fill the at least the portion of the disposable set (40) for disinfection, and wherein either enough concentrate remains in the first container to further prepare dialysis fluid for a subsequent treatment or a second container (84b) of concentrate is used for the subsequent treatment.

11. The renal failure therapy system of any one of Claims 1 to 10, wherein the control unit (22) is configured to cause the dialysis fluid pump to remove the concentrate from the at least the portion of the disposable set (40) prior to preparing dialysis fluid for a subsequent treatment.

12. The renal failure therapy system of any one of Claims 1 to 11, wherein the disposable set (40) includes a heater/mixing bag (62), and wherein the control unit (22) is programmed to cause the heater/mixing bag to collapse prior to introducing the concentrate into the heater/mixing bag for disinfection.

13. The renal failure therapy system of any one of Claims 1 to 12, which includes a last bag of dialysis fluid formulated differently than dialysis fluid made from the concentrate used for disinfection, and wherein the last bag is provided with enough last bag dialysis fluid for a single treatment or the last bag is provided with enough last bag dialysis fluid for multiple treatments using the same disposable set (40).

14. A renal failure therapy system comprising:
a dialysis fluid pumping unit (20) including a dialysis fluid pump;
a disposable set (40) operable with the dialysis fluid pumping unit such that the dialysis fluid pump can pump dialysis fluid from the disposable set;
a concentrate in fluid communication with the disposable set, wherein the concentrate is used to prepare the dialysis fluid;
a conductivity sensor (132) configured to sense when the concentrate has been introduced to the disposable set; and
a control unit (22) operating the dialysis fluid pump,
**characterized in that** the control unit is configured to:
(i) cause a portion of the concentrate to fill at least a portion of the disposable set prior to treatment, the concentrate operating as a disinfectant to disinfect the at least portion of the disposable set prior to treatment,
(ii) use an output of the conductivity sensor to determine whether the concentrate filled the at least a portion of the disposable set,
(iii) when the concentrate did not fill the at least a portion of the disposable set, cause an alarm to be provided that is indicative of an issue with the concentrate, and
(iv) determine whether the disposable set may be used again via the disinfecting concentrate in (i).

15. The renal failure therapy system of any one of claims 1 to 14, wherein determining whether the disposable set (40) may be used again via the disinfecting concentrate includes incrementing a counter each time the concentrate is used as a disinfectant and comparing the counter to a limit.

## Patentansprüche

1. Ein Niereninsuffizienz-Therapiesystem (10), welches Folgendes umfasst:
eine Dialyseflüssigkeitspumpeneinheit (20) mit einer Dialyseflüssigkeitspumpe;
ein Einwegset (40), das mit der Dialyseflüssigkeitspumpeneinheit betrieben werden kann, so dass die Dialyseflüssigkeitspumpe Dialyseflüssigkeit aus dem Einwegset pumpen kann;
ein Konzentrat in Flüssigkeitsverbindung mit dem Einwegset, wobei das Konzentrat zur Herstellung der Dialyseflüssigkeit verwendet wird;
einen Leitfähigkeitssensor (132), der so konfiguriert ist, dass er erkennt, wann das Konzentrat in das Einwegset eingeführt wurde; und
eine Steuereinheit (22), die die Dialyseflüssigkeitspumpe betreibt,
**dadurch gekennzeichnet, dass** die Steuereinheit konfiguriert ist, um:
(i) zu veranlassen, dass ein Teil des Konzentrats zumindest einen Teil des Einwegsets zwischen den Behandlungen füllt, wobei das Konzentrat als Desinfektionsmittel wirkt, so dass dasselbe Einwegset für mehrere Behandlungen mit der Dialyseflüssigkeitspumpeneinheit verwendet werden kann,
(ii) eine Ausgabe des Leitfähigkeitssensors zu verwenden, um zu bestimmen, ob das Konzentrat den mindestens einen Teil des Einwegsets gefüllt hat,
(iii) wenn das Konzentrat den mindestens einen Teil des Einwegsets nicht gefüllt hat, einen Alarm auszulösen, der auf ein Problem mit dem Konzentrat hinweist, und
(iv) zu bestimmen, ob das Einwegset über das desinfizierende Konzentrat in (i) wieder verwendet werden darf.

2. Das Niereninsuffizienz-Therapiesystem nach Anspruch 1, wobei die Steuereinheit (22) so konfiguriert ist, dass sie die Dialyseflüssigkeitspumpe veranlasst, den Teil des Konzentrats zu pumpen, um den mindestens einen Teil des Einwegsets (40) zwischen den Behandlungen zu füllen.

3. Das Niereninsuffizienz-Therapiesystem nach Anspruch 1 oder 2, wobei es sich bei dem Niereninsuffizienz-Therapiesystem um ein Peritonealdialysesystem und bei der Dialysierflüssigkeitspumpe um einen Peritonealdialysecycler handelt.

4. Das Niereninsuffizienz-Therapiesystem nach einem der Ansprüche 1 bis 3, wobei mindestens ein Teil des Einwegsets (40), welches das Konzentrat zwischen den Behandlungen aufbewahrt, eine Pumpkassette (42) umfasst, die mit der Dialyseflüssigkeitspumpe betrieben werden kann, und mindestens eine Leitung (50, 60, 72) in Flüssigkeitsverbindung mit der Pumpkassette enthält, wobei die Pumpkassette eine Patientenleitung (50) enthält und wobei (i) die Pumpkassette so konfiguriert ist, dass sie zwischen den Behandlungen an ein distales Ende der Patientenleitung angeschlossen werden kann, oder (ii) eine Kappe vorgesehen ist, um das distale Ende der Patientenleitung (50) zwischen den Behandlungen abzudecken.

5. Das Niereninsuffizienz-Therapiesystem nach einem der Ansprüche 1 bis 4, wobei das Konzentrat Dextrose enthält.

6. Das Niereninsuffizienz-Therapiesystem nach einem der Ansprüche 1 bis 5, das einen Vorrat an für die Behandlung geeignetem Wasser enthält, und bei dem die Steuereinheit (22) so konfiguriert ist, dass sie das Konzentrat mit dem für die Behandlung geeigneten Wasser mischt, um die Dialyseflüssigkeit zu bilden.

7. Das Niereninsuffizienz-Therapiesystem nach Anspruch 6, wobei das Einwegset (40) einen Behälter (66) zum Sammeln von für die Behandlung geeignetem Wasser enthält und wobei der Sammelbehälter das Konzentrat zwischen den Behandlungen nicht aufnimmt.

8. Das Niereninsuffizienz-Therapiesystem nach Anspruch 6 oder 7, wobei das Konzentrat ein erstes Konzentrat ist und ein zweites Konzentrat enthält und wobei die Steuereinheit (22) so konfiguriert ist, dass sie das erste und das zweite Konzentrat mit dem für die Behandlung geeigneten Wasser mischt, um die Dialyseflüssigkeit zu bilden.

9. Das Niereninsuffizienz-Therapiesystem nach Anspruch 8, wobei das zweite Konzentrat zur Desinfektion einer zweiten Konzentratleitung (78) des Einwegsets (40) zwischen den Behandlungen verwendet wird.

10. Das Niereninsuffizienz-Therapiesystem nach einem der Ansprüche 1 bis 9, wobei das Konzentrat in einem ersten Behälter (84a) in einer solchen Menge bereitgestellt wird, dass nach der Verwendung des Konzentrats zur Herstellung von Dialyseflüssigkeit für die Behandlung genügend Konzentrat übrig bleibt, um zumindest den Teil des Einwegsets (40) für die Desinfektion zu füllen, und wobei entweder genügend Konzentrat in dem ersten Behälter verbleibt, um Dialyseflüssigkeit für eine nachfolgende Behandlung weiter herzustellen, oder ein zweiter Behälter (84b) mit Konzentrat für die nachfolgende Behandlung verwendet wird.

11. Das Niereninsuffizienz-Therapiesystem nach einem der Ansprüche 1 bis 10, wobei die Steuereinheit (22) so konfiguriert ist, dass sie die Dialyseflüssigkeitspumpe veranlasst, das Konzentrat vor der Zubereitung der Dialyseflüssigkeit für eine nachfolgende Behandlung aus dem mindestens einen Teil des Einwegsets (40) zu entfernen.

12. Das Niereninsuffizienz-Therapiesystem nach einem der Ansprüche 1 bis 11, wobei das Einwegset (40) einen Heiz-/Mischbeutel (62) enthält und wobei die Steuereinheit (22) so programmiert ist, dass sie den Heiz-/Mischbeutel zum Kollabieren bringt, bevor das Konzentrat zur Desinfektion in den Heiz-/Mischbeutel eingeführt wird.

13. Das Niereninsuffizienz-Therapiesystem nach einem der Ansprüche 1 bis 12, das einen letzten Beutel mit Dialyseflüssigkeit enthält, die anders formuliert ist als die Dialyseflüssigkeit, die aus dem zur Desinfektion verwendeten Konzentrat hergestellt wurde, und wobei der letzte Beutel mit ausreichend Dialyseflüssigkeit für eine einzelne Behandlung oder der letzte Beutel mit ausreichend Dialyseflüssigkeit für mehrere Behandlungen unter Verwendung desselben Einwegsets (40) versehen ist.

14. Ein Niereninsuffizienz-Therapiesystem, welches Folgendes umfasst:
eine Dialyseflüssigkeitspumpeneinheit (20) mit einer Dialyseflüssigkeitspumpe;
ein Einwegset (40), das mit der Dialyseflüssigkeitspumpeneinheit betrieben werden kann, so dass die Dialyseflüssigkeitspumpe Dialyseflüssigkeit aus dem Einwegset pumpen kann;
ein Konzentrat in Flüssigkeitsverbindung mit dem Einwegset, wobei das Konzentrat zur Herstellung der Dialyseflüssigkeit verwendet wird;
einen Leitfähigkeitssensor (132), der so konfiguriert ist, dass er erkennt, wann das Konzentrat in das Einwegset eingeführt wurde; und
eine Steuereinheit (22), die die Dialyseflüssigkeitspumpe betreibt,
**dadurch gekennzeichnet, dass** die Steuereinheit konfiguriert ist, um:
(i) zu veranlassen, dass ein Teil des Konzentrats zumindest einen Teil des Einwegsets vor der Behandlung füllt, wobei das Konzentrat als Desinfektionsmittel wirkt, umzumindest einen Teil des Einwegsets vor der Behandlung zu dezifizieren,
(ii) eine Ausgabe des Leitfähigkeitssensors zu verwenden, um zu bestimmen, ob das Konzentrat den mindestens einen Teil des Einwegsets gefüllt hat,
(iii) wenn das Konzentrat den mindestens einen Teil des Einwegsets nicht gefüllt hat, einen Alarm auszulösen, der auf ein Problem mit dem Konzentrat hinweist, und
(iv) zu bestimmen, ob das Einwegset über das desinfizierende Konzentrat in (i) wieder verwendet werden darf.

15. Das Niereninsuffizienz-Therapiesystem nach einem der Ansprüche 1 bis 14, wobei die Bestimmung, ob das Einwegset (40) über das Desinfektionskonzentrat wieder verwendet werden darf, das Erhöhen eines Zählers jedes Mal, wenn das Konzentrat als Desinfektionsmittel verwendet wird, und den Vergleich des Zählers mit einem Grenzwert einschließt.

## Revendications

1. Système de traitement pour insuffisance rénale (10) comprenant :
une unité de pompage de fluide de dialyse (20) comportant une pompe à fluide de dialyse ;
un ensemble jetable (40) pouvant fonctionner avec l'unité de pompage de fluide de dialyse de sorte que la pompe à fluide de dialyse peut pomper le fluide de dialyse à partir de l'ensemble jetable ;
un concentré en communication fluidique avec l'ensemble jetable, dans lequel le concentré est utilisé pour préparer le fluide de dialyse ;
un capteur de conductivité (132) configuré pour détecter le moment où le concentré a été introduit dans l'ensemble jetable ; et
une unité de commande (22) faisant fonctionner la pompe à fluide de dialyse,
**caractérisé en ce que** l'unité de traitement est configurée pour :
(i) amener une partie du concentré à remplir au moins une partie de l'ensemble jetable entre les traitements, le concentré agissant comme un désinfectant permettant au même ensemble jetable d'être utilisé pour des traitements multiples avec l'unité de pompage de fluide de dialyse,
(ii) utiliser une sortie du capteur de conductivité pour déterminer si le concentré remplit au moins une partie de l'ensemble jetable,
(iii) lorsque le concentré n'a pas rempli l'au moins une partie de l'ensemble jetable, déclencher une alarme indiquant un problème avec le concentré, et
(iv) déterminer si l'ensemble jetable peut être réutilisé via le concentré désinfectant en (i).

2. Système de traitement pour insuffisance rénale selon la revendication 1, dans lequel l'unité de commande (22) est configurée pour amener la pompe à fluide de dialyse à pomper la partie du concentré pour remplir l'au moins la partie de l'ensemble jetable (40) entre les traitements.

3. Système de traitement pour insuffisance rénale selon la revendication 1 ou 2, dans lequel le système de traitement pour insuffisance rénale est un système de dialyse péritonéale et l'unité de pompage de fluide de dialyse est un cycleur de dialyse péritonéale.

4. Système de traitement pour insuffisance rénale selon l'une quelconque des revendications 1 à 3, dans lequel l'au moins une partie de l'ensemble jetable (40) contenant le concentré entre les traitements comporte une cassette de pompage (42) pouvant fonctionner avec la pompe à fluide de dialyse et au moins une conduite (50, 60, 72) en communication fluidique avec la cassette de pompage, dans lequel la cassette de pompage comporte une ligne de patient (50) et dans lequel (i) la cassette de pompage est configurée pour se connecter à une extrémité distale de la ligne de patient entre les traitements ou (ii) un capuchon est prévu pour recouvrir l'extrémité distale de la ligne de patient (50) entre les traitements.

5. Système de traitement pour insuffisance rénale selon l'une quelconque des revendications 1 à 4, dans lequel le concentré comprend du dextrose.

6. Système de traitement pour insuffisance rénale selon l'une quelconque des revendications 1 à 5, qui comporte une alimentation en eau appropriée pour le traitement, et dans lequel l'unité de commande (22) est configurée pour mélanger le concentré avec de l'eau appropriée pour le traitement pour former le fluide de dialyse.

7. Système de traitement pour insuffisance rénale selon la revendication 6, dans lequel l'ensemble jetable (40) comporte un récipient (66) pour accumuler de l'eau rendue appropriée pour le traitement, et dans lequel le récipient d'accumulation ne reçoit pas le concentré entre les traitements.

8. Système de traitement pour insuffisance rénale selon la revendication 6 ou 7, dans lequel le concentré est un premier concentré et qui comporte un second concentré, et dans lequel l'unité de commande (22) est configurée pour mélanger les premier et second concentrés avec de l'eau rendue appropriée pour le traitement afin de former le fluide de dialyse.

9. Système de traitement pour insuffisance rénale selon la revendication 8, dans lequel le second concentré est utilisé pour désinfecter une seconde conduite de concentré (78) de l'ensemble jetable (40) entre les traitements.

10. Système de traitement pour insuffisance rénale selon l'une quelconque des revendications 1 à 9, dans lequel le concentré est fourni dans un premier récipient (84a) en une quantité de sorte qu'après utilisation du concentré pour préparer le fluide de dialyse pour le traitement, il reste suffisamment de concentré pour remplir l'au moins la partie de l'ensemble jetable (40) pour la désinfection, et dans lequel soit suffisamment de concentré reste dans le premier récipient pour préparer davantage le fluide de dialyse pour un traitement ultérieur, soit un second récipient (84b) de concentré est utilisé pour le traitement ultérieur.

11. Système de traitement pour insuffisance rénale selon l'une quelconque des revendications 1 à 10, dans lequel l'unité de commande (22) est configurée pour amener la pompe à fluide de dialyse à retirer le concentré d'au moins la partie de l'ensemble jetable (40) avant de préparer le fluide de dialyse pour un traitement ultérieur.

12. Système de traitement pour insuffisance rénale selon l'une quelconque des revendications 1 à 11, dans lequel l'ensemble jetable (40) comporte une poche chauffante/mélangeur (62), et dans lequel l'unité de commande (22) est programmée pour provoquer l'affaissement de la poche chauffante/du mélangeur avant d'introduire le concentré dans la poche chauffante/le mélangeur pour la désinfection.

13. Système de traitement pour insuffisance rénale selon l'une quelconque des revendications 1 à 12, qui comporte une dernière poche de fluide de dialyse formulé différemment du fluide de dialyse fabriqué à partir du concentré utilisé pour la désinfection, et dans lequel la dernière poche contient suffisamment de fluide de dialyse dans la dernière poche pour un traitement unique ou la dernière poche contient suffisamment de fluide de dialyse dans la dernière poche pour des traitements multiples à l'aide du même ensemble jetable (40).

14. Système de traitement pour insuffisance rénale comprenant :
une unité de pompage de fluide de dialyse (20) comportant une pompe à fluide de dialyse ;
un ensemble jetable (40) pouvant fonctionner avec l'unité de pompage de fluide de dialyse de sorte que la pompe à fluide de dialyse peut pomper le fluide de dialyse à partir de l'ensemble jetable ;
un concentré en communication fluidique avec l'ensemble jetable, dans lequel le concentré est utilisé pour préparer le fluide de dialyse ;
un capteur de conductivité (132) configuré pour détecter le moment où le concentré a été introduit dans l'ensemble jetable ; et
une unité de commande (22) faisant fonctionner la pompe à fluide de dialyse,
**caractérisé en ce que** l'unité de commande est configurée pour :
(i) amener une partie du concentré à remplir au moins une partie de l'ensemble jetable avant traitement, le concentré agissant comme un désinfectant pour désinfecter l'au moins une partie de l'ensemble jetable avant le traitement,
(ii) utiliser une sortie du capteur de conductivité pour déterminer si le concentré remplit l'au moins une partie de l'ensemble jetable,
(iii) lorsque le concentré n'a pas rempli l'au moins une partie de l'ensemble jetable, déclencher une alarme indiquant un problème avec le concentré, et
(iv) déterminer si l'ensemble jetable peut être réutilisé via le concentré désinfectant en (i).

15. Système de traitement pour insuffisance rénale selon l'une quelconque des revendications 1 à 14, dans lequel le fait de déterminer si l'ensemble jetable (40) peut être réutilisé via le concentré désinfectant comporte l'incrémentation d'un compteur chaque fois que le concentré est utilisé comme désinfectant et la comparaison du compteur à une limite.
